# EUROPÄISCHE PATENTSCHRIFT

(11) **EP 1 651 286 B1**
(45) Veröffentlichungstag und Bekanntmachung des Hinweises auf die Patenterteilung: **30.12.2015**
(21) Anmeldenummer: 03817899.2
(22) Anmeldetag: 08.08.2003
(51) Int. Cl.: A61L 27/56

(54) **VERFAHREN ZUM IMPRÄGNIEREN EINES PORÖSEN KNOCHENERSATZMATERIALS**
METHOD FOR IMPREGNATING A POROUS BONE SUBSTITUTE MATERIAL
PROCEDE D'IMPREGNATION D'UNE MATIERE POREUSE SOUS FORME D'OS ARTIFICIEL

(43) Veröffentlichungstag der Anmeldung: 03.05.2006
(73) Patentinhaber: Synthes GmbH, 4436 Oberdorf (CH)
(72) Erfinder: HOERGER, Flavio, CH-8022 Zürich (CH); STOLL, Thierry, CH-2554 Meinisberg (CH)
(74) Vertreter: Carpmaels & Ransford LLP
(86) Internationale Anmeldenummer: PCT/CH2003/000537
(87) Internationale Veröffentlichungsnummer: WO 2005/014068

(56) Entgegenhaltungen:
- EP-A- 0 361 896
- EP-A- 0 470 393
- EP-A- 1 230 942
- WO-A-02/15950
- WO-A-97/46202
- FR-A- 2 815 021
- US-A- 4 529 511

## Beschreibung

Die Erfindung bezieht sich auf ein Verfahren zum Imprägnieren eines porösen Knochenersatzmaterials gemäss dem Oberbegriff des Patentanspruchs 1 sowie auf eine Vorrichtung zur Imprägnierung eines porösen Knochenersatzmaterials gemäss dem Oberbegriff des Patentanspruchs 11.

Aus der WO02/068010 MUSCHLER ist eine Vorrichtung bekannt, in welcher ein Knochenmarkaspirat mit einem porösen Knochenersatzmaterial mechanisch vermischt werden kann. Dabei wird das Knochenmarkaspirat mittels zweier Spritzen durch das Knochenersatzmaterial gepresst oder gesogen, so dass dieses vom Knochenmarkaspirat umspült wird. Bei diesem bekannten Verfahren wird somit die in den Poren des Knochenersatzmaterials befindliche Luft nicht entfernt.

Eine weitere Vorrichtung zur Imprägnierung eines porösen, biokompatiblen Knochenersatzkörpers ist aus der US 6,049,026 MUSCHLER bekannt. Diese bekannte Vorrichtung umfasst eine Kammer zur Aufnahme des Knochenersatzkörpers sowie oberhalb der Kammer einen ersten Behälter zur Speicherung eines Imprägniermittels und unterhalb der Kammer einen zweiten Behälter zur Aufnahme des durch die Kammer mit dem Knochenersatzkörper fliessenden Imprägniermittels. Durch Öffnen eines ersten, zwischen dem ersten Behälter und der Kammer angeordneten Ventils fliesst das Imprägnierungsmittel in die Kammer mit dem Knochenersatzkörper. Sobald die Kammer gefüllt ist, wird ein zweites, zwischen der Kammer und dem zweiten Behälter angeordnetes Ventil geöffnet, so dass das Imprägniermittel durch eine unterhalb des Knochenersatzkörpers angeordnete Membran in den zweiten Behälter abfliessen kann. Nachteilig an dieser bekannten Vorrichtung ist, dass die Kammer in ihrem Volumen nicht veränderbar ist, so dass für verschieden grosse Knochenersatzkörper Kammern in mehreren Grössen vorhanden sein müssen.

WO 02/15950 offenbart eine Vorrichtung und ein Verfahren zur Herstellung eines Knochenersatzmaterials. Das Verfahren besteht darin, dass ein biokompatibler, offenporiger Körper einem Vakuum ausgesetzt wird und osteoinduktive und/oder osteogene Substanzen in fliessfähiger Form mittels des in den Poren des Körpers erzeugten Vakuums in diese Poren aufgesaugt werden.

Neben den üblicherweise angewendeten Verfahren, bei welchen solche Formkörper aus porösen Knochenersatzmaterialien in eine Schale mit patienteneigenem Blut gelegt werden, zeigen auch diese bekannte Vorrichtung die Nachteile, dass bei der Imprägnierung nicht gewährleistet ist, dass
- feste Blutbestandteile, wie z.B. Blutplättchen oder andere Zellen bis in den Kern des Implantates vordringen können. Die Blutzellen werden in der Randzone des Implantates konzentriert und festgehalten (Filterwirkung des Knochenersatzmateriales); und dass
- die ganze Luft aus dem Implantat entfernt werden kann. Lufteinschlüsse bilden eine Barriere für das Einwachsen von Knochen und behindern den Umbau von resorbierbaren Knochenersatzmaterialien.

Hier will die Erfindung Abhilfe schaffen. Der Erfindung liegt die Aufgabe zugrunde, ein Verfahren zum Imprägnieren eines porösen Knochenersatzmaterials zu schaffen, welches die in den Poren des Knochenersatzmaterials befindliche Luft entfernt und durch das gewünschte Imprägniermittel ersetzt.

Die Erfindung löst die gestellte Aufgabe mit einem Verfahren zum Imprägnieren eines porösen Knochenersatzmaterials, welches die Merkmale des Anspruchs 1 aufweist sowie mit einer Vorrichtung zur Imprägnierung eines porösen Knochenersatzmaterials, welche die Merkmale des Anspruchs 11 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank des erfindungsgemässen Verfahrens die in den Poren des Knochenersatzmateriales vorhandene Luft entfernt werden kann und das gewünschte Imprägnierungsmittel in die Poren eindringen kann.

In einer bevorzugen Ausführung wird die Luft oder das Gas durch die bezüglich des Schwerkraftvektors oben an der Kammer angeordnete Öffnung abgesogen, so dass Imprägniermittel durch die Schwerkraft in der Kammer zurückgehalten wird.

Das Knochenersatzmaterial kann in Form eines Blocks, vorzugsweise in Form eines Würfels, Zylinders, Hohlzylinders, Scheibe, Keils, Kegels, Kegelstumpfes oder einer Kugel vorliegen oder in einer anderen Ausführung des Verfahrens in Form eines Granulates vorliegen.

In wiederum einer anderen Ausführung umfasst das Imprägniermittel osteoinduktive und/oder osteogene Substanzen, insbesondere Körperzellen, Knochenmark oder Knochenmarkbestandteilen, Blut oder Blutbestandteilen oder eine Kombination davon.

In einer weiteren Ausführung wird durch das in Schritt d) des Verfahrens gemäss Anspruch 1 erzeugte Vakuum der in der Kammer anfänglich herrschende Umgebungsdruck von 1 Bar auf unter 0,9 Bar, vorzugsweise auf unter 0,6 Bar erniedrigt.

In wiederum einer weiteren Ausführung des Verfahrens wird durch das in Schritt d) des Verfahrens gemäss Anspruch 1 erzeugte Vakuum der in der Kammer anfänglich herrschenden Umgebungsdruck von 1 Bar bis auf unter 0,2 Bar, vorzugsweise auf unter 0,1 Bar erniedrigt.

Das Einführen des Imprägniermittels kann
- im Unterdruck-Durchfluss durch Ansaugen des Imprägniermittels durch eine der beiden Öffnungen in die Kammer;
- im Überdruck-Durchfluss durch Einpressen des Imprägniermittels durch eine der beiden Öffnungen in die Kammer; oder
- durch mehrfache Wiederholung der Verfahrensschritte d) und e) gemäss Anspruch 1 erfolgen.

In einer bevorzugten Ausführungsform ist das Innenvolumen V der Kammer variabel, wobei die Kammer vorzugsweise aus einem kreiszylindrischen Behälter mit Innengewinde und einem dazu passenden Deckel mit Aussengewinde besteht, so dass das Innenvolumen V der Kammer durch ein mehr oder weniger tiefes Einschrauben des Deckels in den kreiszylindrischen Behälter variierbar ist. Damit ist der Vorteil erreichbar, dass eine einzige Kammergrösse genügt, um verschieden grosse Implantate darin aufnehmen zu können.

Dadurch dass die Kammer derart mit einem porösen Knochenersatzmaterial gefüllt wird, dass dessen Gesamtvolumen v kleiner ist als das Innenvolumen V der Kammer ist, kann das Knochenersatzmaterial teilweise oder vorzugsweise vollständig in das Imprägniermittel eingetaucht werden.

In einer anderen Ausführung der Kammer ist das Knochenersatzmaterial derart in einem Implantat aus Metall und/oder Kunststoff untergebracht, dass es mindestens teilweise mit der Oberfläche des Implantates kommuniziert.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht einer Kammer zur Durchführung des erfindungsgemässen Verfahren zusammen mit einer Spritze;
Fig. 2 eine Explosionsdarstellung einer Kammer zur Durchführung des erfindungsgemässen Verfahrens;
Fig. 3 einen Längsschnitt durch eine Kammer zur Durchführung des erfindungsgemässen Verfahrens;
Fig. 4 eine Aufsicht auf die in Fig. 3 dargestellte Kammer zur Durchführung des erfindungsgemässen Verfahrens.

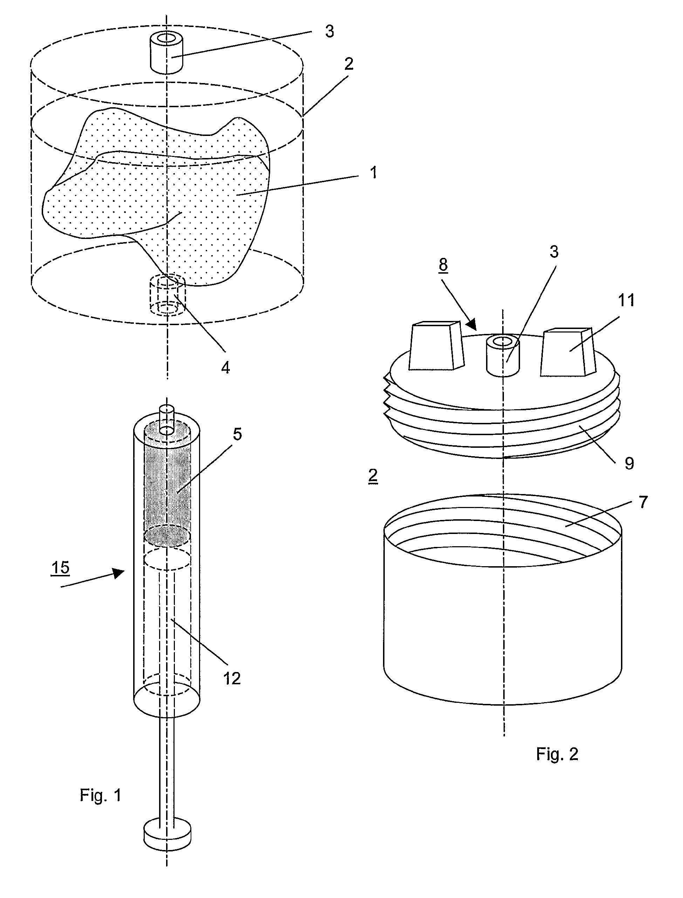

Fig. 1 zeigt eine Kammer 2 mit einem im Hohlraum eingeschlossenen, aus Knochenersatzmaterial 1 bestehenden Körper. Die Kammer 2 hat zwei Öffnungen 3;4, welche mit einer Spritze 15 luftdicht verbindbar sind. Als Imprägniermittel 5 kann die Spritze 15 in ihrem Hohlraum osteoinduktive und/oder osteogene Substanzen, insbesondere Körperzellen, Knochenmark und/oder Knochenmarkbestandteile, Blut und/oder Blutbestandteile enthalten. Die hier dargestellte Kammer 2 dient zusammen mit einer Spritze 15 dazu, das die folgenden Schritte umfassende erfindungsgemässe Verfahren aufzuführen:
A) Eine mit Imprägniermittel 5 gefüllte Spritze 15 wird vorzugsweise an die untere der beiden Öffnungen 4 der Kammer 2 angeschlossen. Die obere Öffnung 3 bleibt unverschlossen. Beide Öffnungen 3;4 sind als Luer-Öffnungen mit einem konisch durchbohrten, mit der Kammer 2 verbundenen Anschlusstück 21;25 ausgestaltet. Das Imprägniermittel 5 wird nun mittels des Kolbens 12 durch die untere Öffnung 4 in die Kammer 2 eingespritzt, so dass der Block aus porösem Knochenersatzmaterial 1 vom Imprägniermittel 5 umgeben wird und in diesem teilweise, vorzugsweise ganz untertaucht;
B) Nun wird die obere Öffnung 3 verschlossen;
C) Danach wird der Kolben 12 der Spritze 5 wieder zurückgezogen, so dass in der Kammer 2 ein Unterdruck oder Vakuum entsteht. Durch das Vakuum wird nun die in den Poren des Knochenersatzmaterial 1 vorhandene Luft zum Expandieren gebracht, so dass sie aus den Poren in das umgebende Imprägniermittel 5 austritt. Da es sich um ein geschlossenes System handelt, wird das Imprägniermittel 5 nur teilweise durch die Kolbenbewegung abgesaugt. Dies ist auch nur möglich, wenn noch Luft in der Kammer 2 enthalten ist. Bei einer grossen Anzahl von Imprägniermitteln 5 kommt hinzu, dass sie adhärente Eigenschaften aufweisen, somit auf der Oberfläche des Knochenersatzmaterials 1 anhaften und durch die Kolbenbewegung nicht abgesaugt werden;
D) In einem nächsten Schritt wird der Kolben 12 der Spritze 5 wieder in die ursprüngliche Position gedrückt, so dass das Vakuum in der Kammer 2 aufgehoben wird. Der vom Imprägniermittel 5 umgebene Block aus Knochenersatzmaterial 1 nimmt nun in seinen Poren natürlich keine Luft sondern Imprägniermittel 5 auf, so dass eine Imprägnierung des Knochenersatzmaterial 1 stattfindet. Die mittels der Spritze 5 durchführbare Evakuierung/Aufhebung des Vakuums in der Kammer 2 kann mehrere Male wiederholt werden, um den Imprägnierungsgrad zu erhöhen. Das Imprägniermittel 5 wird durch dessen Adhäsion und durch die Kappilarwirkung der Struktur des porösen Knochenersatzmaterials 1 eher ins Innere aufgenommen als Luft.

Eine andere Ausführung des Verfahrens besteht darin, dass nachdem die erste, mit Imprägniermittel 5 gefüllte Spritze 15 an eine der zwei Öffnungen 3;4 angekuppelt worden ist, eine zweite, ungefüllte Spritze (nicht gezeichnet) an die andere Öffnung 3;4 angekuppelt wird, und durch Zurückziehen des Kolbens der Hohlraum der Kammer 2 evakuiert wird und zugleich das Imprägniermittel 5 durch den entstehenden Unterdruck aus dem Hohlraum in der Spritze 15 in den Hohlraum der Kammer 2 gesaugt wird. Die Luft in den Poren des Knochenersatzmateriales 1 tritt aus den Poren aus. Anschliessend wird die Luft durch Einschieben des Kolbens der zweiten Spritze wieder in die Kammer 2 gefördert, so dass das Vakuum im Hohlraum der Kammer 2 wieder aufgehoben wird und das Imprägniermittel 5 in die Poren des Knochenersatzmaterials eindringen kann. Falls erforderlich kann nun der Kolben einer der Spritzen zurückgezogen werden und die Kammer 2 erneut evakuiert werden. Die Verfahrensschritte des Evakuierens und des Aufhebens des Vakuums können auf diese Weise einfach wiederholt werden, bis die Poren im Knochenersatzmaterial 1 ausreichend entlüftet und mit Imprägniermittel 5 gefüllt sind.

Wiederum eine andere Ausführung des Verfahrens besteht darin, dass die zweite Spritze (nicht gezeichnet) zur Vergrösserung des Vakuums angewendet wird. Diese zweite Spritze kann - da sie nicht mit Imprägniermittel 5 gefüllt ist - ein erheblich grösseres Volumen aufweisen als die erste, mit Imprägniermittel 5 gefüllte Spritze 15.

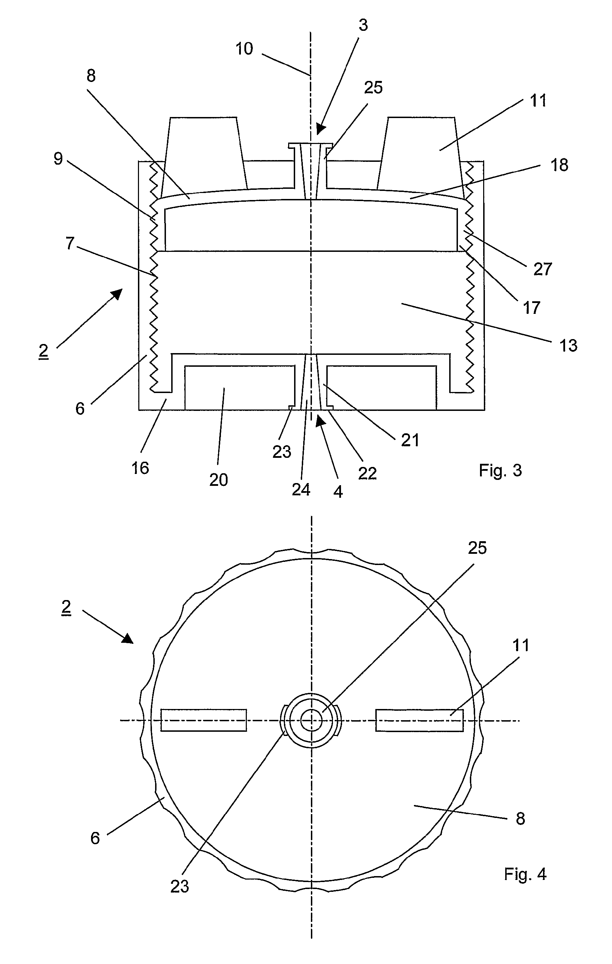

In den Fig. 2 bis 4 ist eine Ausführungsform der Kammer 2 dargestellt, welche einen zur Zentralachse 10 koaxialen, kreiszylindrischen Behälter 6 mit einem koaxial befestigbaren Deckel 8 umfasst. Der Behälter 6 weist in seinem Hohlraum 13 ein zur Zentralachse 10 koaxiales Innengewinde 7 auf, so dass der Deckel 8, welcher auf seiner seitlichen Mantelfläche ein zum Innengewinde 7 komplementäres Aussengewinde 9 aufweist, mit dem Behälter 6 lösbar verbindbar ist. Das Innengewinde 7 erstreckt sich über die gesamte axiale Länge des Hohlraumes 13 im Behälter 6. Das Aussengewinde 9 an der seitlichen Mantelfläche des Deckels 8 erstreckt sich über die gesamte axiale Länge des Deckels 8, so dass der Deckel 8 auch in den Hohlraum 13 des Behälter 6 einschraubbar ist und somit das freie Volumen im Hohlraum 13 des Behälters 6 soweit verkleinert werden kann, bis das vordere Ende 17 des rohrförmigen Fortsatzes 27 am Deckels 8 am Boden 16 des Behälters 6 ansteht. Das Innengewinde 7 sowie das Aussengewinde 9 sind als mehrgängige Gewinde ausgestaltet. Der Boden 16 des Behälters 6 ist mit einer Vertiefung 20 versehen, wo konzentrisch zur Zentralachse 10 ein zentral durchbohrtes Anschlusstück 21 angeordnet ist. Die Zentralbohrung 24 des Anschlusstückes 21 bildet die Öffnung 4. Das Anschlusstück 21 ist an seinem freien Ende 22 mit zwei radial vorstehenden Nocken 23 versehen, so dass eine zum Anschlusstück 21 komplementär ausgestaltete, an ihrer Öffnung ein Innengewinde aufweisende Standardspritze 15 über das Anschlusstück 21 schraubbar ist (Luer-Verbindung). Anstelle der Luer-Verbindung können beispielsweise auch die Zentralbohrungen 24 der Anschlusstücke 21;25 derart konisch ausgebildet sein, dass eine Spritze 15 mit Standardkonus in eine der Zentralbohrungen 24 der Anschlusstücke 21;25 einführbar und mittels der Konusverbindung fixierbar ist. Die Anschlüsse können auch so gewählt werden, dass verschiedene Adapter luftdicht angeschlossen werden können. Durch die Anordnung des Anschlusstückes 21 in der Vertiefung 20 derart, dass das Anschlusstück 21 nicht über den Boden 16 des Behälters 6 vorsteht, wird erreicht, dass der Behälter 6 auf den Boden gestellt werden kann, ohne zu kippen. Analog dazu ist aussen an der Deckplatte 18 des Deckels 8 ein zweites, konzentrisch zur Zentralachse 10 durchbohrtes Anschlusstück 25 angeordnet. Die Anschlusstücke 21 und 25 sind identisch ausgestaltet. Zur Erleichterung des Verschraubens oder Aufschraubens der Kammer 2 sind auf der Aussenseite der Deckplatte 18 am Deckel 8 zwei axial vorstehende Erhebungen 11 und an der äusseren Mantelfläche des Behälters 6 axial verlaufende Rillen 26 angebracht.

## Patentansprüche

1. Verfahren zum Imprägnieren eines porösen Knochenersatzmaterials (1) mit einem flüssigen Imprägniermittel (5),
**dadurch gekennzeichnet, dass**
a) das Knochenersatzmaterial (1) mit dem Gesamtvolumen v in eine Kammer (2) mit dem Innenvolumen V > v mit zwei verschliessbaren Öffnungen (3,4) eingeschlossen ist oder wird;
b) das Imprägniermittel (5) in die Kammer (2) eingeführt wird bis das Knochenersatzmaterial (1) mindestens teilweise im Imprägniermittel (5) untergetaucht ist;
c) eine der beiden Öffnungen (3;4) verschlossen wird;
d) die Kammer (2) über die andere, unverschlossene Öffnung (4;3) mindestens teilweise evakuiert wird, so dass die in den Poren des Knochenersatzmaterials (1) enthaltene Luft mindestens teilweise daraus entweicht; und
e) das in der Kammer (2) erzeugte Vakuum durch Zuführung von Luft oder eines Gases durch eine der Öffnungen (3;4) wieder aufgehoben wird; so dass
f) das Imprägniermittel in die Poren des im Imprägniermittel eingetauchten Knochenersatzmaterials (1) eindringt.

2. Verfahren nach Anspruch 1, **dadurch gekennzeichnet, dass** unter Schritt d) die Luft oder das Gas durch die bezüglich des Schwerkraftvektors oben an der Kammer (2) angeordnete Öffnung (3) abesogen wird.

3. Verfahren nach Anspruch 1 oder 2, **dadurch gekennzeichnet, dass** das Knochenersatzmaterials (1) in Form eines Blocks, vorzugsweise in Form eines Würfels, Zylinders, Hohlzylinders, Scheibe, Keils, Kegels, Kegelstumpfes oder einer Kugel vorliegt.

4. Verfahren nach Anspruch 1 oder 2, **dadurch gekennzeichnet, dass** das Knochenersatzmaterials (1) in Form eines Granulates vorliegt.

5. Verfahren nach einem der Ansprüche 1 bis 4, **dadurch gekennzeichnet, dass** das Imprägniermittel osteoinduktive und/oder osteogene Substanzen, insbesondere Körperzellen, Knochenmark oder Knochenmarkbestandteilen, Blut oder Blutbestandteilen oder eine Kombination dieser umfasst.

6. Verfahren nach einem der Ansprüche 1 bis 5, **dadurch gekennzeichnet, dass** das in Schritt d) erzeugte Vakuum den in der Kammer (2) anfänglich herrschenden Umgebungsdruck von 1 Bar auf unter 0,9 Bar, vorzugsweise auf unter 0,6 Bar erniedrigt.

7. Verfahren nach Anspruch 6, **dadurch gekennzeichnet, dass** das in Schritt d) erzeugte Vakuum den in der Kammer (2) anfänglich herrschenden Umgebungsdruck von 1 Bar bis auf unter 0,2 Bar, vorzugsweise auf unter 0,1 Bar erniedrigt.

8. Verfahren nach einem der Ansprüche 1 bis 7, **dadurch gekennzeichnet, dass** das Imprägniermittel (5) im Unterdruck-Durchfluss durch Ansaugen durch eine der beiden Öffnungen(3;4) in die Kammer (2) eingeführt wird.

9. Verfahren nach einem der Ansprüche 1 bis 7, **dadurch gekennzeichnet, dass** das Imprägniermittel (5) im Überdruck-Durchfluss durch Einpressen durch eine der beiden Öffnungen(3;4) in die Kammer (2) eingeführt wird.

10. Verfahren nach einem der Ansprüche 1 bis 7, **dadurch gekennzeichnet, dass** das die Verfahrensschritte d) und e) mehrfach wiederholt werden.

11. Vorrichtung umfassend eine Kammer (2) zur Imprägnierung eines porösen Knochenersatzmaterials (1) mit einem Imprägniermittel (5), wobei die Kammer (2) zwei verschliessbare Öffnungen (3,4) aufweist und ein veränderbares Innenvolumen V besitzt,
**dadurch gekennzeichnet, dass**
die Kammer (2) aus einem kreiszylindrischen Behälter (6) mit einem Hohlraum (13), einem im Hohlraum (13) angeordneten Innengewinde (7) und einem dazu passenden Deckel (8) mit Aussengewinde (9) besteht und dass das Innenvolumen V der Kammer (2) durch ein mehr oder weniger tiefes Einschrauben des Deckels (8) in den kreiszylindrischen Behälter (6) variierbar ist, wobei
die Vorrichtung zusätzlich eine Spritze (15) umfasst, wobei jede Öffnung (3,4) luftdicht mit der Spritze (15) verbindbar ist.

12. Verwendung einer Vorrichtung nach Anspruch 11 zur Durchführung des Verfahrens nach einem der Ansprüche 1 bis 10.

## Claims

1. Method for impregnating a porous bone substitute material (1) with a liquid impregnating agent (5),
**characterized in that**
a) the bone substitute material (1), with the total volume v, is enclosed in a chamber (2), with the internal volume V > v and with two closeable openings (3, 4);
b) the impregnating agent (5) is introduced into the chamber (2) until the bone substitute material (1) is at least partially immersed in the impregnating agent (5) ;
c) one of the two openings (3; 4) is closed;
d) the chamber (2) is at least partially evacuated via the other, unclosed opening (4; 3), such that the air contained in the pores of the bone substitute material (1) escapes at least partially therefrom; and
e) the vacuum generated in the chamber (2) is cancelled again by delivering air or a gas through one of the openings (3; 4), such that
f) the impregnating agent penetrates into the pores of the bone substitute material (1) immersed in the impregnating agent.

2. Method according to Claim 1, **characterized in that**, during step d), the air or the gas is aspirated through the opening (3) arranged on the top of the chamber (2) in relation to the gravitational force vector.

3. Method according to Claim 1 or 2, **characterized in that** the bone substitute material (1) is present in the form of a block, preferably in the form of a cube, cylinder, hollow cylinder, disc, wedge, cone, truncated cone or a sphere.

4. Method according to Claim 1 or 2, **characterized in that** the bone substitute material (1) is present in the form of granules.

5. Method according to one of Claims 1 to 4, **characterized in that** the impregnating agent comprises osteoinductive and/or osteogenic substances, in particular body cells, bone marrow or bone marrow constituents, blood or blood constituents, or a combination thereof.

6. Method according to one of Claims 1 to 5, **characterized in that** the vacuum generated in step d) reduces the atmospheric pressure initially prevailing in the chamber (2) from 1 bar to below 0.9 bar, preferably to below 0.6 bar.

7. Method according to Claim 6, **characterized in that** the vacuum generated in step d) reduces the atmospheric pressure initially prevailing in the chamber (2) from 1 bar to below 0.2 bar, preferably to below 0.1 bar.

8. Method according to one of Claims 1 to 7, **characterized in that** the impregnating agent (5) is introduced into the chamber (2) by being aspirated through one of the two openings (3; 4) in the negative pressure flow.

9. Method according to one of Claims 1 to 7, **characterized in that** the impregnating agent (5) is introduced into the chamber (2) by being injected through one of the two openings (3; 4) in the positive pressure flow.

10. Method according to one of Claims 1 to 7, **characterized in that** method steps d) and e) are repeated a number of times.

11. Device comprising a chamber (2) for impregnating a porous bone substitute material (1) with an impregnating agent (5), wherein the chamber (2) has two closeable openings (3, 4) and a variable internal volume V,
**characterized in that**
the chamber (2) is composed of a circular cylindrical container (6) with a hollow space (13), an internal thread (7) arranged in the hollow space (13), and a matching lid (8) with an external thread (9), and **in that** the internal volume V of the chamber (2) is variable by screwing the lid (8) into the circular cylindrical container (6) to a greater or lesser depth, wherein
the device additionally comprises a syringe (15), wherein each opening (3, 4) can be connected in an airtight manner to the syringe (15).

12. Use of a device according to Claim 11 for carrying out the method according to one of Claims 1 to 10.

## Revendications

1. Procédé d'imprégnation d'un matériau poreux de substitution osseux (1) comprenant un agent d'imprégnation fluide (5),
**caractérisé en ce que**
a) le matériau de substitution osseux (1) de volume total v est enfermé dans une chambre (2) de volume interne V > v avec deux ouvertures refermables (3, 4) ;
b) l'agent d'imprégnation (5) est introduit dans la chambre (2) jusqu'à ce que le matériau de substitution osseux (1) soit submergé au moins en partie dans l'agent d'imprégnation (5) ;
c) l'une des deux ouvertures (3 ; 4) est fermée ;
d) la chambre (2) est au moins en partie évacuée par le biais de l'autre ouverture non fermée (4 ; 3), de telle sorte que l'air contenu dans les pores du matériau de substitution osseux (1) s'en échappe au moins en partie ; et
e) le vide généré dans la chambre (2) est à nouveau supprimé par alimentation en air ou en un gaz à travers l'une des ouvertures (3 ; 4) ; de telle sorte que
f) l'agent d'imprégnation pénètre dans les pores du matériau de substitution osseux (1) plongé dans l'agent d'imprégnation.

2. Procédé selon la revendication 1, **caractérisé en ce que** l'air ou le gaz est aspiré à l'étape d) par l'ouverture (3) disposée par rapport au vecteur de force de gravité en haut de la chambre (2).

3. Procédé selon la revendication 1 ou 2, **caractérisé en ce que** le matériau de substitution osseux (1) se présente sous forme de bloc, de préférence sous forme de cube, de cylindre, de cylindre creux, de disque, de coin, de cône, de tronc de cône ou de sphère.

4. Procédé selon la revendication 1 ou 2, **caractérisé en ce que** le matériau de substitution osseux (1) se présente sous la forme d'un granulé.

5. Procédé selon l'une quelconque des revendications 1 à 4, **caractérisé en ce que** l'agent d'imprégnation comprend des substances ostéo-inductives et/ou ostéogènes, en particulier des cellules corporelles, de la moelle ou des composants de la moelle, du sang ou des composants sanguins, ou d'une combinaison de ceux-ci.

6. Procédé selon l'une quelconque des revendications 1 à 5, **caractérisé en ce que** le vide généré à l'étape d) abaisse la pression ambiante régnant au début dans la chambre (2) de 1 bar à moins de 0,9 bar, de préférence à moins de 0,6 bar.

7. Procédé selon la revendication 6, **caractérisé en ce que** le vide généré à l'étape d) abaisse la pression ambiante régnant au début dans la chambre (2) de 1 bar à moins de 0,2 bar, de préférence à moins de 0,1 bar.

8. Procédé selon l'une quelconque des revendications 1 à 7, **caractérisé en ce que** l'agent d'imprégnation (5) est introduit dans un flux soumis à une dépression par aspiration à travers l'une des deux ouvertures (3 ; 4) à l'intérieur de la chambre (2).

9. Procédé selon l'une quelconque des revendications 1 à 7, **caractérisé en ce que** l'agent d'imprégnation (5) est introduit dans un flux soumis à une surpression par pressage à travers l'une des deux ouvertures (3 ; 4) à l'intérieur de la chambre (2).

10. Procédé selon l'une quelconque des revendications 1 à 7, **caractérisé en ce que** les étapes de procédé d) et e) sont répétées plusieurs fois.

11. Dispositif comprenant une chambre (2) pour l'imprégnation d'un matériau de substitution osseux (1) avec un agent d'imprégnation (5), la chambre (2) présentant deux ouvertures refermables (3, 4) et possédant un volume interne variable V,
**caractérisé en ce que**
la chambre (2) se compose d'un récipient cylindrique circulaire (6) avec une cavité (13), un filetage interne (7) disposé dans la cavité (13), et d'un couvercle (8) s'ajustant à celui-ci avec un filetage externe (9), et le volume interne V de la chambre (2) peut être varié par un vissage plus ou moins profond du couvercle (8) dans le récipient cylindrique circulaire (6),
le dispositif comprenant en outre une seringue (15), chaque ouverture (3, 4) pouvant être connectée de manière étanche à l'air à la seringue (15).

12. Utilisation d'un dispositif selon la revendication 11 pour mettre en oeuvre le procédé selon l'une quelconque des revendications 1 à 10.
